# EUROPEAN PATENT APPLICATION

(11) **EP 1 186 665 A1**
(43) Date of publication of application: **13.03.2002**
(21) Application number: 01203404.7
(22) Date of filing: 10.09.2001
(51) Int. Cl.: C12N 15/62, G01N 33/574, C07K 14/16, C07K 14/01, C12N 15/34, C12N 1/21, C12N 15/87, A61K 38/16

(54) **A delivery method for the tumor specific apoptosis inducing activity of apoptin**

(30) Priority: 08.09.2000 EP 00203115; 11.09.2000 EP 00203147; 28.09.2000 US 236117 P
(71) Applicant: Leadd B.V., 2333 AL Leiden (NL)
(72) Inventor: Noteborn, Mathieu Hubertus Maria, 2352 EH Leiderdorp (NL); Voorhoeve, Pieter Mathijs, 1091 TS Amsterdam (NL); Zhang, Ying-Hui, 2334 ET Leiden (NL); Leliveld, Sirik Rutger, 2311 TS Leiden (NL)
(74) Representative: Prins, Adrianus Willem

(57) **Abstract**

The invention relates to the field of apoptosis. The invention provides novel therapeutic substances, for example novel therapeutic proteinaceous compounds that can contain apoptin alone or jointly with other proteinaceous protein or protein fragments, especially in those cases when cells are derailed such as cancer-, auto-immune-derived cells.

## Description

### Brief description of the invention

The invention relates to a method for inducing apoptosis specifically in transformed or malignant cells by introduction of protein with apoptin (vp3) like activity into these cells.

### Background of the invention

Apoptosis is an active and programmed physiological process for eliminating superfluous, altered or malignant cells (Earnshaw, 1995, Duke et al., 1996). Apoptosis is characterized by shrinkage of cells, segmentation of the nucleus, condensation and cleavage of DNA into domain-sized fragments, in most cells followed by internucleosomal degradation. The apoptotic cells fragment into membrane-enclosed apoptotic bodies. Finally, neighbouring cells and/or macrophages will rapidly phagocytose these dying cells (Wyllie et al., 1980, White, 1996).
The apoptotic process can be initiated by a variety of regulatory stimuli (Wyllie, 1995, White 1996, Levine, 1997). Changes in the cell survival rate play an important role in human pathogenesis, e.g. in cancer development and autoimmune diseases, which is caused by enhanced proliferation but also by decreased cell death (Kerr et al., 1994, Paulovich, 1997). A variety of chemotherapeutic compounds and radiation have been demonstrated to induce apoptosis in tumor cells, in many instances via wild-type p53 protein (Thompson, 1995, Bellamy et al., 1995, Steller, 1995, McDonell et al., 1995).

Many tumors, however, acquire a mutation in p53 during their development, often correlating with poor response to cancer therapy. Certain transforming genes of tumorigenic DNA viruses can inactivate p53 by directly binding to it. Examples of such agents are the E6 protein from oncogenic subtypes of the Human Papiloma Virus and the large T antigen of the tumor DNA virus SV40 it (Teodoro, 1997).

Another example of the emergence of a strong resistance to various apoptosis-inducing chemotherapeutic agents in (leukemic) tumors is the association of a high expression level of the proto-oncogene Bcl-2 or Bcr-abl with reduced sensitivity of these tumors to therapy (Hockenberry 1994, Sachs and Lotem, 1997).

Apoptin (also called vp3; the terms may be used interchangeable herein) is a small protein derived from chicken anemia virus (CAV; Noteborn and De Boer, 1995, Noteborn et al., 1991, Noteborn et al., 1994; 1998a). By means of transient transfection using plasmids encoding apoptin it was shown that apoptin can induce apoptosis in human malignant and transformed cell lines, but not in non-transformed human cell cultures. In vitro, also by means of transient transfections, apoptin fails to induce programmed cell death in normal lymphoid, dermal, epidermal, endothelial and smooth-muscle cells. However, when normal cells are transformed they become susceptible to apoptosis induced by apoptin. In normal cells, apoptin was found predominantly in the cytoplasm, whereas in transformed or malignant cells i.e. characterized by hyperplasia, metaplasia or dysplasia, it was located in the nucleus (Danen-van Oorschot, 1997 and Noteborn, 1996).

Long-term expression of apoptin in normal human fibroblasts revealed that apoptin has no toxic or transforming activity in these cells (Danen-van Oorschot, 1997 and Noteborn, 1996). The fact that apoptin does not induce apoptosis in normal human cells, at least not in vitro, suggests that a toxic effect of apoptin treatment in vivo will be very low. Noteborn and Pietersen (1998) and Pietersen et al. (1999) have provided evidence that adenovirus expressed apoptin does not have an acute toxic effect in vivo, whereas in nude mice it has a strong anti-tumor activity. Further evidence of a lack of toxicity in vivo comes from transgenic mice which express apoptin from an MHC-I promoter and which have no observable abnormalities (Noteborn and Zhang, 1998).

Of importance in the treatment of tumors that have become resistant to chemo or radiation therapy is that apoptin-induced apoptosis occurs in the absence of functional p53 (Zhuang et al., 1995a), and cannot be blocked by Bcl-2, Bcr-abl (Zhuang et al., 1995), or the Bcl-2-associating protein BAG-1 (Danen-Van Oorschot, 1997a, Noteborn, 1996). In addition, it appears, that even premalignant, minimally transformed cells, may be sensitive to the death-inducing effect of apoptin (Noteborn and Zhang, 1998).

Thus, to further enlarge the array of therapeutic anti-cancer or anti-auto-immune-disease compounds available in the art, additional therapeutic proteins are desired. The invention provides novel therapeutic substances, for example novel therapeutic proteinaceous compounds that can contain apoptin alone or jointly with other proteinaceous substances or protein fragments, especially in those cases when cells are derailed such as cancer-derived or auto-immune-derived cells.
In a first embodiment, the invention provides a proteinaceous apoptin-like containing substance that can induce apoptosis in a tumor-specific way (tumor-specific apoptosis). Apoptin protein exerts its tumor-specific cytotoxicity when administered to cells. Preferably said apoptin or functional equivalent or functional fragment thereof is provided with a means to deliver apoptin to a cell. A functional equivalent or functional fragment is any equivalent or fragment having the same kind of activity and specificity as apoptin, possibly in different amounts. An example of a functional fragment is, as disclosed herein within the experimental part, a fragment which comprises the amino acids 66-121. It is clear to a person skilled in the art that functional equivalents can be obtained for example by pointmutations. More preferably said means to deliver apoptin to a cell comprises a protein transduction system (HIV TAT protein). Even more preferably said Apoptin is further provided with a fusion protein or tag. Preferably the apoptin protein is non-denatured.

In particular, the detailed description provides evidence that micro-injection of either an apoptin fusion protein, such as non-denatured Maltose-binding-protein (MBP)-apoptin fusion proteins or a His-tagged apoptin protein, both produced in E. coli cells and purified using affinity-purification, result in apoptosis induction of human tumorigenic/transformed cells but not of normal human primary cells. Furthermore, the his-tagged protein was purified under denaturing conditions and subsequently allowed to refold. The proteins showed specific activity, showing that both non-denatured protein and refolded protein can be used.

In yet another embodiment the invention provides a nucleic acid encoding apoptin according to the invention. Furthermore the invention provides a vector comprising a nucleic acid according to the invention. Examples of such a vector are given in the experimental part given herein, examples are MBP-vp3, pVp3H6 or pMalTBVp3dN66 and so on. Furthermore, the invention provides a host cell comprising a nucleic acid or a vector according to the invention. Examples comprise a prokaryotic cell such as E.coli, as described in the experimental part herein.

The invention also describes that the activity and behavior of the recombinant proteins is similar to the activity of apoptin protein produced by transcription and translation of the apoptin DNA without protein fusion or tag. This indicates that the apoptin DNA is not necessary for apoptin like activity other than as a template for transcription by the cell. The invention shows furthermore that the presence of a fusion protein on the N-terminus of apoptin, or of a six his-tag on the C-terminus, does not disturb the specificity or the activity of apoptin. Also the specific localization to the nucleus in transformed cells is observed with recombinant apoptin protein, indicating that the localization of apoptin protein can be used as a diagnostic marker for a transformed/tumorigenic phenotype of tissue samples or cells cultured in vitro.

The invention also describes that due to its ability to differentiate between normal and transformed cells that recombinant apoptin protein harbors activity for the destruction of tumor cells, or other hyperplasia, metaplasia or dysplasia, with minimal or no toxicity to normal tissue. Moreover, apoptin protein or derivatives of it such as apoptin-fusion proteins will also be effective against tumors which have become resistant to (chemo)-therapeutic induction of apoptosis, due to the lack of functional p53 and (over)-expression of Bcl-2 and other apoptosis-inhibiting agents.

This invention also describes another example of an effective anti-tumor therapy based on apoptin-derived proteinaceous substances. To eradicate a tumor, it is imperative that all cells of the tumor, including its potential mini-metastases, are reached by an anti-tumor agent. Until now this has been a bottleneck in the development of an efficient anti-tumor therapy based on gene delivery.

The invention describes a method allowing direct introduction of apoptin protein into cells achieved in vitro and in vivo by coupling this effector protein (henceforth referred to as the cargo) to a protein transduction domain. The first description of the capability of certain proteins to cross cell membranes was given independently by Green and Loewenstein (1988) and Frankel and Pabo (1988), for the HIV TAT protein. Henceforth it was shown that synthetic peptides containing the amino acids 48 to 60 derived from the HIV TAT protein could transduce into cells (Vives et al., 1997). This ability can be conferred in trans by chemical crosslinking the TAT-derived protein transduction domain to the cargo protein, enabling proteins as large as 120 kDa to be delivered intra-cellularly, in vitro as well as in vivo (Fawell et al., 1994). Other transduction domains have been described in the Antennapedia protein from Drosophila melanogaster (Derossi et al., 1998) as well as in other homeodomain proteins, the Herpes Simplex Virus vp22 protein (Elliott et al., 1999), and several synthetic peptides (Lindgren et al., 2000). The HIV-TAT-mediated process does not depend on endocytosis and is not mediated through a cellular receptor. This explains the remarkable universality that is seen; the proteins can be transduced into all cells tested thus far (Schwarze and Dowdy, 2000). An efficient method based on the HIV TAT peptide has been developed to make recombinant proteins that can be transduced both in vitro and in vivo. Significantly, when administered in vivo, all tissues, including the brain, can be targeted with a recombinant protein (Schwarze, 1999) using this system.

Another class of delivery method is based on the hydrophobic core region of signal peptides (Hawiger, 1999). The cellular import of fusions or conjugations between these transduction domains and a cargo are concentration and temperature sensitive. It is, however, cell type independent and a whole range of cells have been shown to be susceptible to internalization of cargo mediated by this class of transduction domains.

In general for the transduction protein technology the drawback is that it has not yet been possible to target a specific (tumor) cell compartment with this system. The lack of tumor specific targeting has thus far prevented the development of an efficient anti-tumor therapy, which uses protein transduction.

In a preferred embodiment, the present invention describes a method, which circumvents this drawback. Transduced cells, which take up apoptin-derived protein are only undergoing cell death when they are of a transformed or malignant nature and stay alive when they are normal. This means that the use of apoptin as part of a transduction-capable proteinaceous substance will make it a potent anti-tumor agent.

The invention describes another way to introduce (recombinant) apoptin protein into cells, which is by fusion of the protein with a ligand. Receptor mediated internalization then results in the uptake of the fusion protein. Examples for such apoptin fusion proteins are based on the Epidermal Growth Factor (EGF), and apoptin-fusion proteins containing ligands binding to the hormone receptors such as thyroid receptors.

All these methods hinge on the activity of the recombinant or purified cargo-protein in the target cell.
In particular, the invention shows that apoptin protein produced in various ways retains its specific tumor killing ability, and thus opens the way to combine the generalized delivery of protein transduction with the specific anti-tumor activity of apoptin, which results in a new method by which transformed or malignant cells can be eradicated.

Thus, the invention discloses several examples of apoptin-derived proteinaceous substances such as MBP apoptin cross-linked to a transduction domain such as TAT can be applied, and also his-tagged apoptin protein containing a transduction domain as a fusion can be applied, either as a non-denatured protein or in denatured renatured format. It is not relevant how a proteinaceous substance, comprising apoptin and a transduction domain or any another delivery compound, is obtained (for example, chemically cross-linked or as produced as a fusion protein) but it is important that, as disclosed herein within the experimental part, that apoptin retains, in all cases, its tumor-specific apoptosis activity.

Furthermore, the invention discloses other transduction domains (for example (single-chain) antibodies), where all share the capacity to introduce the apoptin protein into tumor cells and normal cells alike. Fusion to a ligand may specifically target apoptin to one cell type, but the tumor specificity of apoptin will be pivotal for therapeutic applications with minimal collateral damage to normal cells. As an example, EGF-targetted apoptin could be introduced into all EGF-receptor expressing cells. Apoptin will, however, destroy only the tumor cells. The invention provides the application of cell-permeable protein as a drug being much more safe in the long term than gene-therapy approaches possibly causing genetic alterations resulting in diseases such as cancer.

Therefore, the invention provides use of a proteinaceous substance comprising apoptin or functional equivalent or functional fragment thereof for induction of tumor-specific apoptosis. A functional equivalent or functional fragment is any equivalent or fragment having the same kind of activity and specificity as apoptin, possibly in different amounts. Preferably said proteinaceous substance further comprises a means to deliver said apoptin to a cell. More preferably said means to deliver apoptin to a cell comprises a protein transduction system (e.g. HIV TAT protein). Even more preferably said Apoptin is further provided with a fusion protein or tag. Preferably the apoptin protein is non-denatured. Typically, said induction of tumor-specific apoptosis is p53-independent.

Use as provided by the invention is particularly useful from a therapeutic viewpoint. The invention provides herewith a pharmaceutical composition comprising a proteinaceous substance capable of providing tumor specific apoptosis. The said proteinaceous substance comprises apoptin or functional equivalent or functional fragment thereof. More preferably, the proteinaceous substance further comprises a means to deliver said apoptin to a cell, a fusion protein and/or a tag. The proteinaceous substance according to the invention can be either produced as a non-denatured compound or refolded into an active state.

A pharmaceutical composition according to the invention is in particular provided for the induction of apoptosis, for example wherein said apoptosis is p53-independent, for the treatment of a disease where enhanced cell proliferation or decreased cell death is observed. These compositions are important for new treatments, but also for diagnosis of diseases with aberrancies in the apoptotic process, such as cancer and (auto-) immune diseases.

Thus in yet another embodiment, the invention provides the use of a proteinaceous substance comprising apoptin or functional equivalent or functional fragment thereof capable of providing apoptosis for the preparation of a pharmaceutical composition for the treatment of a disease where enhanced cell proliferation or decreased cell death is observed. preferably said proteinaceous substance further comprises a means to deliver said apoptin to a cell. More preferably said means to deliver apoptin to a cell comprises a protein transduction system (e.g. HIV TAT protein). Even more preferably said Apoptin is further provided with a fusion protein or tag. Preferably the apoptin protein is non-denatured. Preferably, said induction of tumor-specific apoptosis is p53-independent and even more preferably said said disease comprises cancer or auto-immune disease.

In the field of diagnosis the invention provides a method for detecting the presence of cancer cells or cells that are cancer prone in a sample of cells comprising providing cells in said sample with a proteinaceous substance capable of providing tumor specific apoptosis according to the invention, culturing said cells and determining for example, the percentage of apoptosis of cells in said sample or determining the localization of the proteinaceous substance. The said proteinaceous substance comprises apoptin or functional equivalent or functional fragment thereof. More preferably, the proteinaceous substance further comprises a means (a protein transduction system, for example TAT) to deliver said apoptin to a cell, a fusion protein and/or a tag. The invention further provides evidence that the proteinaceous substance according to the invention can be either non-denatured or refolded.

The invention will be explained in more detail in the following description, which is not limiting the invention.

### Detailed description of the invention

The invention provides a system to produce and deliver intracellularly (recombinant) proteinaceous substances comprising apoptin or functional equivalent or functional fragments there of, or recombinant proteinaceous substances with apoptin-like activity.

The invention further provides for the addition of further optional modular peptides. This can include an epitope tag, allowing easy detection and immunoprecipitation without direct steric hindrance of associations of apoptin with cellular proteins.

The invention provides or describes all steps needed for the production of recombinant apoptosis inducing agent apoptin, or derivatives of apoptin that have a similar tumor specificity. The recombinant protein can be produced in E. coli, insect cells by means of a baculovirus-based vector, or in yeast strains (such as Pichia pastoris).

The invention provides evidence that the apoptin or apoptin-like proteinaceous substance does not need to be folded properly in the producer cell, enabling the production of recombinant apoptin or apoptin-like proteinaceous substances in transgenic plant cells, for mass production.

The invention proposes a modified metal affinity tag for optimal purification of the recombinant protein. By using a double His-tag next to the transduction domain, a significantly better binding to Nickel beads is achieved, resulting in the possibility to wash the recombinant apoptin or apoptin-like proteinaceous substances under very stringent conditions resulting in an optimal purification of apoptin or apoptin-like proteinaceous substances.

The invention describes the use of a transduction domain fused to recombinant apoptin protein. This domain allows the recombinant protein to pass through the cellular membrane. This domain can consist of a transduction domain derived from HIV TAT, or of any other known transduction domain.

The invention describes the use of CAV-derived VP3 (apoptin) proteinaceous substance as part of a fusion protein, or derivatives of apoptin proteinaceous substances that are selected on their ability to specifically induce apoptosis in tumor cells. The invention also provides a therapy for cancer, autoimmune diseases or related diseases which is based on apoptin or apoptin-like proteinaceous substances.

The invention also provides a method to remove aberrant cells in their first stages of transformation and premalignant lesions.

The invention describes a research tool based on apoptin or apoptin-like proteinaceous substances to unravel apoptosis and transformation pathways or as diagnostics for determining the tumorigenic status of patient material.

### Experimental part

### 1. Production and purification of MBP-apoptin (MBP-vp3) protein

### 1.1 MBP-vp3 expression construct

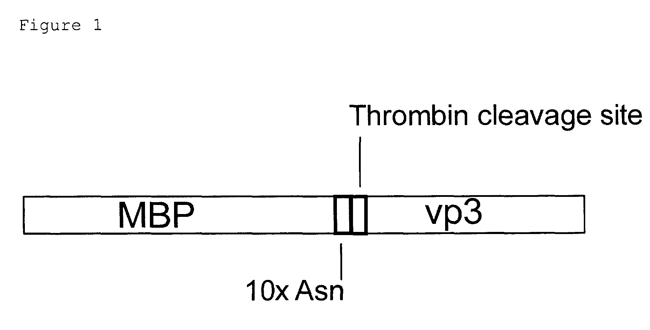

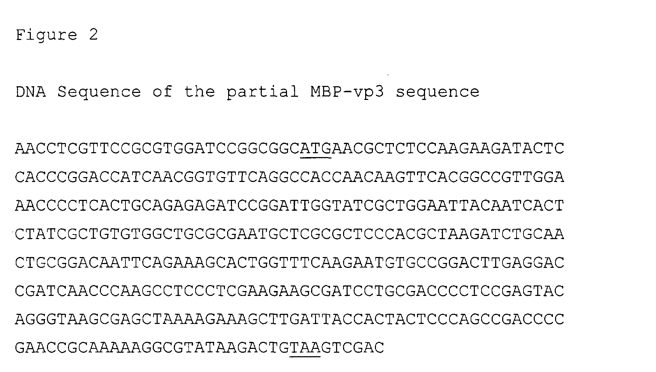

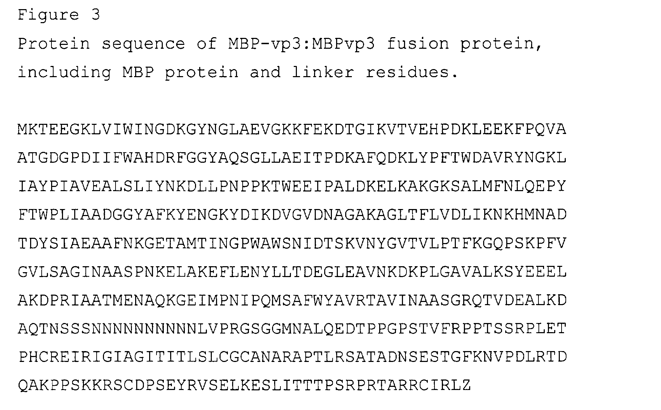

The vp3 gene was fused in frame in a bacterial expression vector encoding the Maltose-binding protein (MBP), a 10 Asn linker and a Thrombin-cleavage site. The expression system is based on a modified pMal-c2 plasmid vector (New England Biolabs, USA), in which the factor Xa site has been replaced by a thrombin-cleavage site. This modified vector was named pMalTB. A PCR fragment consisting of the complete apoptin or vp3 sequences and at the 5'-end a BamH1 and at the 3'-end a SalI site, was cloned in pMalTB. The resulting fusion product consists of a N-terminal MBP moiety that is separated from the vp3 part by a 10-Asn linker and a thrombin-cleavage site as shown in Figure 1. The DNA and protein sequence of the fusion product is given in Figure 2 and Figure 3, respectively. The resulting plasmid is called pMBP-vp3 and the proteinaceous substance encoded by this plasmid is designated MBP-vp3. The correct sequence of the essential parts of the MBP-vp3 construct was confirmed by means of the Sanger method (Sanger et al., 1973) and carried out by Base-Clear, Leiden, The Netherlands.

### 1.2 Expression and purification of MBP-vp3

The plasmid pMBP-vp3 was transformed in bacteria derived from strain BL21(DE3), and initial expression studies showed that MBP-vp3 protein constitutes roughly 10% of the soluble cytoplasmatic protein, after 3 hours induction with 1mM IPTG. Purification was carried out on amylose beads at pH 7.4 and 1M NaCl. Subsequently, elution in buffer containing 20mM HEPES 8.0, 50mM NaCl, 1mM EDTA, 1mM DTT, 10mM maltose, yielded about 100mg protein per liter of bacterial culture. This purified protein was loaded on a UNO-S1 chromatography column (Biorad), and the fractions that elute at 400-500mM NaCl, 20mM HEPES pH7.4, 1mM EDTA were pooled, dialyzed against PBS and concentrated with Millipore UltraFree spin filters.
The negative control preparation being the Maltose binding protein (MBP) was produced and purified in the way as been described for MBP-vp3.

### 2. Expression and purification of His-tagged apoptin

### 2.1 His-tagged vp3 construct

Vp3 lacking a stop codon was cloned in the NdeI site and NotI site of the IPTG-inducible bacterial expression plasmid pET22b, which provides in frame a 6-histidine tag and a stop codon. The essential regions of the final pVp3H6 DNA construct were sequenced according to the Sanger method and carried out by Base Clear, Leiden, The Netherlands. The map of the essential regions of pVp3H6 is shown in Figure 4a and the DNA and protein sequence of the his-tagged vp3 (apoptin) region is given in Figure 5 and 6, respectively.

### 2.2 Vp3H6 expression and purification

The Vp3H6 construct was transformed in BL21(DE3) bacteria (Novagen) and a colony was grown at 37°C to an OD600 of ca. 0.6. Expression was then induced by adding 1 mM IPTG and the cells were grown for an additional 3 hrs. After harvesting by centrifugation, the cells were lysed in a Bead-Beater (Biospec Inc.) in lysis buffer (containing 50 mM NaHEPES pH 7.4, 100 mM NaCl, 1 mM EDTA, 1 mM DTT and protein inhibitors (Complete, Boehringer)). The inclusion bodies were harvested by centrifugation and made soluble by suspending in Solubilisation Buffer (containing 50 mM HEPES pH 7.4, 20 mM Glycine, 1 mM EDTA, 10 mM DTT, 8 M Urea). The cleared supernatant was loaded directly on UNO-S12 (Biorad), pre-equilibrated with: 20 mM KPO₄, 5 mM Imidazole, 6 M urea, 1 mM GSH. The Vp3H6 protein was eluted with a NaCl gradient (0-1 M NaCl at 3 ml/min with a total volume of 200 ml). Vp3H6 eluted between 400 and 650 mM NaCl. It was loaded directly on Ni-NTA (Qiagen) (pre-equilibrated in 20 mM KPO₄ pH 7.4, 5 mM Imidazole, 500 mM NaCl, 6 M urea at 4C). Next, the column was washed with 20 mM KPO₄ pH 7.4, 20 mM Imidazole, 500 mM NaCl, 6 M GuHCl. The GuHCl was removed by washing with 20 mM KPO₄ pH 7.0, 400 mM NaCl, 2 mM MgCl2, 1 mM GSH, and Vp3H6 protein eluted with 20 mM KPO₄ pH 7.4, 400 mM NaCl, 500 mM Imidazole, 2 mM MgCl2. The Vp3H6 protein containing peak fractions were pooled and 5 mM EDTA was added to remove Nickel traces. The sample was dialysed (1 volume to 200) to 20 mM KPO₄ pH 6.5, 400 mM NaCl, 2 mM MgCl2, 1 mM DTT. Finally, the Vp3H6 protein was concentrated on Centricon YM3 filters (Millipore) to at least 7 mg/ml.

### 3. Characteristics of purified MBP-vp3 and Vp3H6 protein

### 3.1 Recombinant MBP-vp3 and Vp3H6 form non-covalent multimedia complexes

### Dynamic Light Scattering

All dynamic light scattering (DLS) measurements were recorded on a DynaPro-MS/X (Protein Solutions Inc.), at room temperature. BSA was used as a control (0.5 mg ml⁻¹ in PBS, 0.5 mM EDTA). MBP-vp3was measured at 10 µM (PBS, 0.5 mM EDTA), refolded Vp3H6 at 35 µM (in 20 mM KPO₄ pH 6.5, 400 mM NaCl, 2 mM MgCl₂). Per experiment, between 20 and 40 measurements were collected. Protein molecular weight (kDa) was estimated from hydrodynamic radius (Rh) by: MW (kDa) = [1.68 x Rh]^{2.34}.

### Electron microscopic analysis.

*Biotin labeling.* Fresh MBP-vp3 (20 mg ml⁻¹, 0.1 M NaHCO₃ pH 8.3) was incubated with 5 mM sulfo-NHS-LC-biotin (Molecular Probes Inc.) at room temperature for 3 hours. The reaction was terminated by adding 10 mM ethanolamine. The labeled protein was passed over a PD-10 desalting column (Pharmacia), and equilibrated in 20 mM HEPES pH 7.4, 0.1 mM EDTA, to remove unincorporated label.

*Electron microscopy.* Both labeled and unlabeled MBP-vp3 (30 mg ml⁻¹, in 20 mM HEPES pH 7.4, 0.1 mM EDTA) were filtered over 0.22 µm and adsorbed to a carbon-coated polioform layer grid. Both samples were incubated with concentrated streptavidin-gold conjugate (5 nm) (KPL Inc.) and stained with 3% uranyl acetate. Electron microscopy was performed on Philips TEM 410 transmission electron microscope.

### 3.1.1 MBP-vp3

In *E.* coli, MBP-vp3 (or MBP-Apoptin, the terms are used interchangeably herein) is abundantly expressed in a soluble form. After affinity chromatography on amylose resin and cation exchange chromatography, MBP-vp3migrates as a single species on a size exclusion chromatography column (Superose 6 HR 10/30). Its molecular weight was calculated to be 2.5 ± 0.3 MDa. This feature was found to be independent of protein concentration (0.5 to 25 mg ml⁻¹). Increased ionic strength (2x PBS) and the presence of detergent (0.5% CHAPSO or 1% Triton X-100) do not disrupt the MBP-vp3complex. Its size was corroborated by dynamic light scattering (DLS), which showed only one solute species with an average hydrodynamic radius (Rh) of 17.9 ± 2.0 nm (corresponding to an estimated molecular weight of 2.9 ± 0.6 MDa). The variation in the average diameter of the MBP-vp3particle between different protein batches remained within experimental error (16 to 20 nm). As demonstrated by reducing and non-reducing SDS-PAGE, MBP-vp3is a non-covalently linked complex. In addition to the full-length 58.5 kDa species, three less abundant expression products of 56.7, 53.3 and 50.1 kDa consistently co-purify with MBP-vp3. All three are detectable by anti-Apoptin monoclonal antibody (MAb) 111.3 (epitope: residues 18 to 23, Danen-van Oorschot *et al.*, 1997). Since MBP was found to be highly protease-resistant, these products arise from partial C-terminal degradation of the Apoptin moiety. The smallest degradation product is an MBP fusion to an N-terminal fragment of Apoptin of approximately 70 residues. Under native conditions, these products cannot be removed by conventional methods, which demonstrates that the degradation products are still incorporated into the MBP-vp3 complex. In the linker peptide that connects the MBP and Apoptin moieties, there is a thrombin cleavage site. When the fusion protein is digested with thrombin, Apoptin remains part of the complex whereas MBP is released. Hence, all biophysical and functional studies are conducted with intact MBP-vp3. Trace amounts of unfused MBP do not co-elute with MBP-vp3, showing that the Apoptin moiety has a negligible affinity for MBP. We conclude that the multimerization behaviour of MBP-vp3depends, probably, entirely on the Apoptin moiety.
Electron microscopic analysis of purified MBP-vp3 shows a uniform population of globular particles with a diameter of around 20 nm. For identification, MBP-vp3 is labeled with sulfo-NHS-LC-biotin and incubated with a streptavidin-gold conjugate (5 nm gold). Between 5 and 10% of globules show gold binding, while aselective binding is less than 0.1%.

### 3.1.2 Vp3H6

Vp3H6 (or Apoptin-H₆, the terms are used interchangeably herein) was expressed at 20 to 40 mg l⁻¹ as inclusion bodies. Reducing growth temperature or induction time, lowering of the IPTG concentration or varying the lysis conditions did not increase its solubility. After solubilization in 8 M urea, Vp3H6 was purified to near homogeneity using cation exchange chromatography. Immediately afterwards, it can be refolded in one step while bound to Ni²⁺-NTA with a net efficiency of around 50%. Supplementing the expression medium with Zn²⁺ did not promote the expression of soluble Vp3H6, nor did it increase its refolding yields. Nevertheless, the presence of certain divalent cations did improve the solubility of purified, refolded Vp3H6. In this respect, Mg²⁺ was the most effective of all cations tested. However, refolded Vp3H6 has only a mild preference for Mg²⁺, when compared to Ca²⁺, Zn²⁺ or Mn²⁺. Hence, we assume that the stabilizing effect of Mg²⁺ relies on aspecific shielding of charged residues.
On Superose 6 HR 10/30, refolded Vp3H6 migrates as a single species with a molecular weight of 400 ± 50 kDa. The particle size is independent of protein concentration (1 to 7 mg ml⁻¹). There are two additional peaks at 30 and 10 kDa, but these do not contain any MAb 111.3-reactive species. The particle size of Vp3H6 is confirmed by DLS, indicating a single solute species with an Rh of 8.7 ± 1.0 nm. The variation in complex size between protein batches remained within experimental error (8 to 10 nm). The apparent molecular weight of Vp3H6 on SDS-PAGE is 18 kDa. There are a number of additional species migrating at around 8, 34 and 48 kDa, which can be detected by MAb 111.3. All of these additional species co-elute with the complex during size exclusion chromatography. The Vp3H6 complex is non-covalent, as shown by non-reducing SDS-PAGE. The smallest MAb 111.3-reactive species corresponds to an N-terminal fragment, which lacks the C-terminal hexahistidine tag. Like in the MBP-vp3complexes, fragments truncated at the C-terminus remain associated and co-purify.

We demonstrate with a range of techniques, that recombinant constructs of Apoptin form multimeric globules of a distinct size. The average sizes of MBP-vp3and Vp3H6 homoglobules suggest a monomer content of 45 and 30 subunits, respectively. However, the presence of an 18-residue linker between MBP and Apoptin in the MBP-vp3 complex will lead to an overestimation of its hydrodynamic radius. Probably both types of Apoptin homoglobules consist of 30 to 40 subunits, which suggests that they are structurally equivalent. Our EM studies indicate that Apoptin multimers have a roughly spherical shape.

Our findings could indicate that Apoptin is active as a multimeric species. We suggest that Apoptin's propensity to form globular multimeric aggregates of a well-defined size is crucial for its biological function. Also other apoptosis associated proteins form multimers (Bax, Apaf-1), and these multimeric complexes probably have a well-defined internal structure. Bax oligomers form pores in the mitochondrial outer membrane, releasing cytochrome c into the cytoplasm (Antonsson *et al*, 2000 and 2001). Apaf-1 activates caspase-9 upon oligomerisation (Cain *et al,* 1999). If Apoptin homoglobules have a well-defined internal structure, they may likewise exhibit structural or enzymatical characteristics.

### 3.2 IMP-vp3 subunits are exchanged between homoglobules

### Fluorescent labeling

Fluorescent labels used: 1,5-IADEANS (IA, dissolved in PBS), fluorescein-maleimide (FM, dissolved in 20 mM Na₃PO₄), pyrene-N-maleimide (PM, dissolved in DMSO) (all: Molecular Probes). A fresh stock solution of label (around 10 mM) was diluted to a final concentration of 1 to 2 mM in 2 ml of dialyzed MBP-vp3 (5 to 10 mg/ml) in PBS, 0.1 mM EDTA. The mixture was incubated overnight in the dark at 4 °C. For IA and FM co-labeling, an equimolar amount of label (1 mM) was used. The reaction was stopped by adding 10 mM DTT. The non-conjugated label was removed by passing the sample twice over a 5 ml P6DG cartridge (Biorad), equilibrated in PBS. The level of label incorporation was determined from: [A_{c}/ε_{label}] x [MW (kDa)/cₚᵣₒₜₑᵢₙ (mg/ml)]. All samples were stored in the dark at 4 °C.

### Fluorescence measurements

Fluorescence emission and excitation spectra were recorded on a Perkin Elmer LS-50B. Settings: 2.5 to 6 nm slit width, 120 nm/min scan speed, final average of 3 separate spectra. Background emission and excitation spectra were recorded of the respective filtered dialysis buffers.

*Intrinsic Tyr fluorescence measurements.* Free L-Tyr (Sigma) was used as a control, diluted in dialysis buffer from a stock solution of 100 mM in 1 M HCl. The refolded Vp3H6 sample was prepared as described before, spectra were first recorded in the presence of 2 mM MgCl₂, 0.1 mM ZnCl₂ and then -after a 2 h incubation at RT- in the presence of 5 mM EDTA. The protein concentration of the native sample was determined at 55 µM. In all cases, λ_{exc} was 280 nm.

*PM* fluorescence. PM-β-ME was used as a control: a stock solution of PM (5 mM) was reacted with a 10x molar excess of β-mercaptoethanol (β-ME). This was first diluted 1:100 in MeOH and then to 1 µM in PBS + 0.1 EDTA. MBP-vp3-PM was also diluted to 1 µM in the same buffer. For time course measurements, MBP-vp3-PM was diluted to 10 µM (0.6 mg/ml) and incubated in the dark at 4 °C.
To compensate for concentration changes due to protein precipitation, all spectra were normalized to the 377 nm fluorescence emission peak, which was not affected by monomer/excimer formation. For PM fluorescence, the λ_{exc} was 341 nm. For Trp-PM FRET, λ_{exc} was 280 nm.

*IA/FM FRET.* For FRET measurements, MBP-vp3-FM was mixed with MBP-vp3-IA at a 1:10 label ratio, after which the total protein concentration was adjusted to 10 µM (0.6 mg/ml). The mixture was then incubated in the dark at 30 °C. After 1, 3, 6 and 24 h, samples were filtered (0.22 µM) and diluted to 1 µM, after which fluorescence spectra were recorded (λ_{exc} = 338 nm). To compensate for protein precipitation, the FRET was expressed as the ratio between the IA emission (485 nm) and the FM emission (518 nm), denoted as F₅₁₈/F₄₈₅.

In order to determine whether the architecture of MBP-vp3 homoglobules is either static or dynamic, a method based on fluorescence resonance energy transfer (FRET), for studying subunit exchange between homoglobules was developed. FRET originates from the electronic interaction between a fluorescent donor probe and a fluorescent or non-fluorescent acceptor. The excited state of the donor can decay by transferring its excitation energy to the acceptor probe. Usually, the effective range of FRET is between 10 and 100 Å (Wu and Brand, 1994). The distance-dependency of FRET is summarised in the Förster radius (R₀), which is defined as the distance at which energy tranfer between a specific donor/acceptor pair is 50% effective. Any useful FRET-based approach to monomer exchange in MBP-vp3 requires site-specific labeling of either the MBP or Apoptin moiety. Since Apoptin has four Cys residues and MBP has none, the Apoptin moiety can -in principal- be labeled selectively.

The solvent exposure of protein sulphydryl groups can be quantitated by reaction with DTNB (Riddles *et al*., 1983). Under native conditions, the DTNB-reactivity of freshly prepared MBP-vp3 is around 65% of one molar equivalent of Cys-HCl. In comparison, purified MBP alone showed no reactivity at all with DTNB. This demonstrates that not more than one Cys residue in Apoptin is solvent exposed. Since C30, C47 and C49 are likely to be buried due their localization in the N-terminus of Apoptin (see further) and when the presence of the Apoptin degradation products is taken into account, it can be argued that the single exposed Cys residue in Apoptin is C90. Therefore, Cys-labeling of MBP-Apoptin is thought to occur at a specific location on the protein surface.
MBP-vp3was labeled with 1,5-IADEANS (IA) and fluorescein-5-maleimide (FM). The IA label can act as a FRET donor to FM with an R₀ of 46 Å, which compares well with the probable dimensions of the Apoptin portion of the MBP-vp3particle (Garzon-Rodriques *et al*., 1997). When MBP-vp3-IA is mixed with MBP-vp3-FM, any exchange of subunits between homoglobules is expected to result in the formation of a new effective FRET contact between an IA and FM label. Therefore, an increase in the ratio between FM and IA fluorescence (F₅₁₈/F₄₈₅) can be interpreted as being the result of subunit exchange. However, prolonged incubation of MBP-vp3at 30°C can lead to the formation of aspecific Apoptin aggregates which are prevented from precipitating by the solubilizing propensity of MBP. This can introduce an error in IA/FM FRET measurements. In order to evaluate the relevance of this effect, MBP-vp3was first labeled with N-(1-pyrene)maleimide (PM). A well-documented feature of pyrene fluorescence is the formation of so-called 'excimer' pairs (Lehrer, 1997; Sahoo *et al*., 2000). Pyrene excimer fluorescence is characterised by a broad emission peak around 465 nm and occurs when an excited pyrene comes into close proximity with a ground-state pyrene (<10 Å). When the pyrene-labeled Apoptin domain in MBP-vp3-PM undergoes progressive aspecific aggregation, this is likely to give rise to an increase in excimer fluorescence (Panse *et al.,* 2000). Therefore, an increase in the monomer/excimer ratio can be regarded as a measure of Apoptin intradomain aggregation. An additional informative property of MBP-vp3-PM stems from the fact that Trp can act as a FRET donor for PM (Johnson *et al.,* 2001).
Since MBP contains eight Trp residues and Apoptin contains none, any aspecific aggregation of Apoptin with MBP is expected to result in a rise in Trp/PM FRET. In proteins, the R₀ of the Trp/PM donor/acceptor pair is between 20 and 30 Å (Wu and Brand, 1994). Since the Trp residues in MBP are evenly distributed throughout the molecule, any increase in Trp/PM FRET is a clear indication of interdomain aggregation (Quiocho *et al.,* 1997).

In the pyrene emission spectrum of fresh MBP-vp3-PM, there is a significant amount of excimer fluorescence, when compared to PM-ME (PM-β-mercaptoethanol). The average PM incorporation level in MBP-vp3is 30%, which shows that -on average- a fraction of the exposed Cys sites are less than 10 Å apart. The ratio of monomer/excimer fluorescence is essentially unchanged after 6 h at 30°C. After 24 h at 30 °C, the excimer fluorescence is increased by about 25% (figure 13B). The initial Trp-PM FRET in MBP-vp3-PM is very small and after 24 h at 30°C, the changes in Trp/PM FRET are negligible. Both lines of evidence indicate that in MBP-vp3, aspecific aggregation only begins to be a detectable event after more than 6 h of incubation at 30°C. Moreover, aggregation takes place only within the Apoptin domain. Also, any increase in F₅₁₈/F₄₈₅ can only be ascribed to subunit exchange exclusively up to 6 h of incubation.
The incorporation levels for MBP-vp3-IA and -FM were 150 and 75%, respectively. Since the fusion protein contains an amount of degradation products that lack C90, 75% incorporation is likely to represent near complete Cys-labeling by FM. A fraction of the IA label reacts with a Lys residue on MBP, which was deduced from a strong Trp/IA FRET in MBP-vp3-IA (data not shown). The R₀ of the Trp/IA couple is 22 Å, which is comparable to that of Trp/PM (Wu and Brand, 1994). So, the Trp/IA FRET can only be caused by the IA label located on MBP and not on Apoptin. Since the distance dependence of FRET is in the order of r⁻⁶, the contribution of MBP-IA to Apoptin-IA/FM FRET is thought to be minimal. Indeed, co-labeled MBP-vp3-IA/FM has no significant Trp/IA→FM FRET, although that was expected in view of the intensity of IA fluorescence. The fact that the IA label is not entirely specific for the Apoptin moiety means that a reliable estimate of the IA/FM distance is not possible, since a fraction of donor label does not effectively participate in FRET. Besides, the observation that MBP-vp3-PM displays clear excimer fluorescence shows that the Cys-labeling sites are well within the R₀ of IA/FM. In all, the heterogeneity of IA-labeling does not affect the relationship between F₅₁₈/F₄₈₅ and subunit exchange in MBP-vp3. Furthermore, after 24 h incubation at 30°C, the Rh of labeled MBP-Apoptin complex was indistinguishable from unlabeled and unincubated MBP-vp3, as was shown by DLS. It was also established that MBP-vp3-IA and MBP-vp3-FM are equally sensitive to denaturation at 30°C.
MBP-vp3-IA was combined with MBP-vp3-FM (10:1 label content) and incubated at 30°C in PBS, in the presence of either 0.5 mM EDTA, 0.1 mM ZnCl₂, 2.5 mM MgCl₂. There is a clear increase in F₅₁₈/F₄₈₅ over the course of 6 h at 30 °C, in PBS/EDTA. This demonstrates that MBP-vp3homoglobules are capable of exchanging either monomers or oligomeric subunits under physiological conditions.
The presence of either Mg²⁺ or Zn²⁺ does little to change the exchange rate. However, the presence of EDTA greatly improves the thermal stability of the fusion protein. To test for the influence of detergents on the exchange rate, labeled MBP-vp3 was assayed in PBS, 0.5 mM EDTA with either 1% CHAPSO or 1% Triton X-100. The increase in F₅₁₈/F₄₈₅ in PBS/EDTA + CHAPSO is only around 30% faster than it is in PBS/EDTA. Clearly, the presence of detergents has relatively little effect on the dynamics of the MBP-Apoptin homoglobule. Nevertheless, CHAPSO is clearly favored over Triton X-100. At the concentration used here, the tendency of Triton X-100 to form micelles probably outweighs interaction with MBP-vp3, more so than in CHAPSO.

Overall, these results indicate that the multimeric MBP-vp3 or Vp3H6 complexes are dynamic complexes.

### 3.4 Binding to ss and ds DNA

### DNA-binding

*DNA-affinity chromatography.* MBP-Apoptin was applied to a ss or dsDNA-cellulose column (1.5 x 2 cm; Pharmacia), equilibrated in 20 mM HEPES pH 7.4, 50 mM NaCl, 1 mM EDTA (or 0.1 mM ZnSO₄ or 2.5 mM MgCl₂). The column was then eluted with a NaCl step gradient (5 CV's per step), 50 to 2000 mM NaCl.

*λDNA fragment elution.* λDNA (cI857, strain 7) (Roche) was digested with *Rsa*I (0.3 µg unit⁻¹, 2 h 30 minutes) (NEB), after which *Rsa*I was deactivated by heating at 65°C for 20 minutes. The digest (10 mM Bis-TrisHCl pH 7.0, 10 mM MgCl₂) was mixed directly with MBP-vp3 (50 µg DNA/mg protein) and incubated on ice for 15 minutes. The MBP-vp3-DNA complex was loaded on an amylose column (0.5 x 1.0 cm), which was eluted with a NaCl step gradient (5 CV's per step), 50 to 2000 mM NaCl. Fractions were desalted using Qiaquick spin filters (Qiagen) and separated on 1.2% agarose.

When Apoptin is expressed *in situ* in transformed cells or introduced via microinjection, Apoptin forms intranuclear aggregates. Noteborn (*et al*., 1994) has suggested that Apoptin associates with heterochromatin in apoptotic cells. We evaluated the DNA-binding of MBP-Apoptin *in vitro* by means of ss and dsDNA-affinity chromatography. At pH 7.4, 50 mM NaCl, *all* MBP-Apoptin binds to both ss and dsDNA adsorbed to cellulose with a binding capacity of 1.5 and 2.2 (mg protein/mg of DNA) respectively. The elution profiles of MBP-vp3 on ss and dsDNA are essentialy the same. In both cases, MBP-vp3 elutes as a heterogeneous species with an optimum around 200 mM NaCl. Supplementing the buffer with either Mg²⁺ or Zn²⁺ does not have any effect on the binding capacity and elution profile of MBP-vp3, nor does it induce specificity for either ss or dsDNA.

Next, we devised a method for detecting any sequence specificity in the DNA-binding properties of MBP-vp3. λDNA was digested with *Rsa*I, to yield a set of blunt-ended DNA fragments ranging in size between 0.1 and 2.5 kb. If MBP-vp3 displays sequence specificity, it is likely to bind certain fragments with a relatively high affinity. MBP-vp3was incubated with the λDNA/*Rsa*I fragment collection, bound to amylose and eluted with a NaCl gradient. Subsequently, the fractions were separated on agarose gel. Clearly, there is no obvious specificity for one or more fragments. MBP-vp3 displays a higher for large fragments, but this is expected to stem from a cooperatibe binding effect. Therefore, we conclude that MBP-vp3 is a general DNA-binding protein without significant sequence-specificity. Furthermore, both the MBP-vp3/DNA and DNA/ MBP-vp3 elution profiles show that a fraction of the MBP-vp3/DNA complexes are resistant to treatment with 2 M NaCl. Apparently, MBP-Apoptin is able to form particularly heterogeneous complexes with DNA, some of which are effectively irreversible.

### 4. Biological activity of MBP-vp3 protein and his-tagged apoptin protein.

### 4.1 Tumor-specific induction of apoptosis by proteinaceous apoptin (vp3) substances

To assay the ability of apoptin protein to specifically induce apoptosis in tumor cells a micro-injection system was set up. Human osteosarcoma-derived Saos-2 tumor cells, Jurkat T cells and normal human diploid VH10 primary cells were cultured on glass cover slips. Jurkat T cells are suspension cells, so they were cultured on glass surfaces pre-coated with the lectin wheat germ agglutinin to cause adherence 1 day prior to microinjection. In addition, we obtained human normal primary mesenchymal stem cells and human normal primary hepatocytes from BioWhittaker, which were cultured no more than 1-3 passages in medium and under conditions recommended by the manufacturer. For the cells from BioWhittaker, we included the following control: microinjection into the nucleus of a DNA plasmid, CMV-FADD, which is a positive control for apoptosis induction. The cells were micro-injected in the cytoplasm with protein MBP-vp3, Vp3H6, or MBP alone at 3 mg/ml using an Eppendorf micro-injector with the injection-pressure condition of 0.5 psi, or in the nucleus in the case of CMV-FADD DNA (50 ng/ul). The cells were co-injected with Dextran-Rhodamine (MW: 70 kDa; Molecular Probes, Leiden, The Netherlands) to be able to later identify injected cells. The cells were incubated at 37°C after injection until the cells were fixed with formaldehyde-methanol-acetone. Presence of MBP-apoptin or his-tagged apoptin protein was determined with immuno-histochemistry with antibodies directed against MBP (monoclonal mouse anti-MBP-clone R29; Zymed Laboratories, Inc. and polyclonal rabbit anti-MBP- clone C18/sc-808; Santa Cruz Biotechnology, Inc.) and/or against apoptin (vp3; anti-VP3C). Presence of FADD was determined with a monoclonal FADD antibody (Transduction Laboratories). Apoptosis was counted as abnormal nuclear morphology after counter-staining with DAPI and examination with an immunofluorescence microscope (Telford et al., 1992).

The results show that both MBP-vp3 and Vp3H6 protein were able to induce rapid apoptosis in human tumor cells (within 3-6 hours, but did not induce apoptosis in normal human cells. The MBP control protein did not induce apoptosis in any of the cell lines under these conditions. In contrast, FADD induced rapid apoptosis in normal cells, confirming that the cells were at least competent to undergo apoptosis, and highlighting the fact that they were strongly resistant to proteinaceous apoptin-induced apoptosis. The fact that both proteinaceous apoptin (vp3) substances can induce apoptosis in human tumor cells lacking p53 implies that both proteinaceous apoptin substances induce apoptosis where known anti-cancer therapies fail. In addition, the fact that apoptin was completely harmless in the notoriously chemotherapeutically sensitive primary liver and stem cells underscores that proteinaceous apoptin should not be toxic in human patients.

Furthermore, the apoptin-characteristic tumor-specific cellular localization was observed for both MBP-vp3 as well as for Vp3H6 protein. In the human tumorigenic Saos-2 cells, both MBP-vp3 and Vp3H6 apoptin proteinaceous substance was predominantly located within the nucleus of pre-apoptotic cells. Somewhat later, these MBP-vp3- and Vp3H6-positive cells underwent apoptosis. In normal non-transformed human VH10 cells, mesenchymal stem cells and hepatocytes, both MBP-vp3 and Vp3H6 are mainly localized in cytoplasmic structures as has been described for apoptin by Danen-Van Oorschot et al. (1997).

In conclusion, exogenous produced proteinaceous apoptin substances, comprising a MBP fusion and/or a (His)6 tag, harbor a tumor-specific apoptosis activity, which is non-toxic for primary human cells and that can be the base of a novel anti-cancer therapy.

### 4.2 Apoptosis induction in the absence of de novo protein production.

The following experiment shows an example of an application of the proteinaceous apoptin substance for studying the tumor-specific apoptosis pathway, which can be induced by apoptin. Saos-2 cells were incubated with transcription inhibitors cycloheximide (20 ug/ml or actinomycin D (10 ug/ml) or translation inhibitors puromycin (10 ug/ml) and emetin (10 ug/ml), or without these inhibitors. The transcription- as well as the translation-inhibitors were obtained from the company Sigma, USA. The inhibition efficacy of the various transcription- and/or translation-inhibitors was proven to be accurate in Saos-2 cells by micro-injecting Saos-2 cells with a plasmid expressing the Green-fluorescence protein (GFP). In all cases no GFP protein could be produced by the Saos-2 cells when they were treated with any of the 4 translation- or transcription-inhibitors. In contrast, the Saos-2 cells micro-injected with the GFP-encoding plasmid produced the GFP in clearly detectable amounts as visualized by direct fluorescence techniques.

For each type of inhibitor, 2 dishes with Saos-2 cells were used. As positive control, Saos-2 cells without inhibitors were also grown. All cell cultures were micro-injected with MBP-vp3 protein (produced and purified as described above) or MBP protein. In all cases when MBP-vp3 was micro-injected the majority of the Saos-2 cells underwent apoptosis 6 hours after micro-injection, whereas cells treated with one of the inhibitors did not undergo apoptosis, even at later time points. Independent of the presence of inhibitors, cells injected with MBP protein did not go into apoptosis.

These results indicate that apoptin-induced apoptosis is not inhibited by the transcription-inhibitors cycloheximide or actinomycin D and not by translation-inhibitors emetine or puromycin. Therefore, one can conclude that apoptin protein can induce apoptosis in human tumor cells without de novo synthesis of cellular proteins.

### 4.4 Tumor-specific apoptosis induction by fluorescein-labelled MBP-VP3.

Fluorescein-labelled MBP-vp3 was prepared as described under point 3.2 of the present application.
In order to determine whether chemical coupling of a substance to proteinaceous Apoptin was possible without altering its tumor-specific death characteristics, we directly labeled MBP-VP3 with a fluorescein moiety and performed microinjection experiments on Saos-2 and VH10 cells as described above. The only difference was in this case, the MBP-VP3 did not need to be stained with antibodies to observe it under fluorescence microscopy.

These experiments show that fluorescein-labeled Apoptin translocates to the nucleus of tumor cells and induces apoptosis with similar kinetics to that of unlabelled Apoptin. Furthermore, fluorescein-labeled Apoptin remained in the cytoplasm of normal cells and did not induce apoptosis. These results show that proteinaceous Apoptin can be readily coupled to a chemical sidegroup and that this coupling does not have to interfere with the tumor-specific functioning of Apoptin. It is likely that other compounds or peptides can be similarly attached to proteinaceous Apoptin, or functional fragments thereof, without loss of function or specificity; such technology could allow a novel means for e.g. tumor-specific nuclear delivery or tumor-specific toxin delivery. The avialabilty of a sidegroup, which does not affect the properties of the apoptin protein, is also used as a diagnostic or research tool. Furthermore, this sidegroup can also be a protein or a peptide, for example, TAT, tumor-specific (single-chain) antibodies or EGF as a delivery means.

### 4.5 Apoptosis induction of the his-tagged NLS-apoptin fragment containing amino acids 1-69.

In the following experiments, we have examined whether a bacterially produced chimeric protein consisting of the N-terminal half of apoptin (amino acids 1-69) with at its N-terminus the nuclear localization signal (amino acids N-terminal-Proline-Proline-Lysine-Lysine-Lysine-Arginine-Lysine-Valine-C-terminal) of SV40 large T antigen and at its C-terminus a histidine tag, also reveals apoptotic activity in human tumor cells.

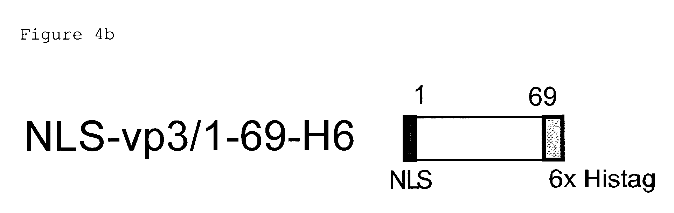

The used expression plasmid encoding the apoptin protein fragment NLS-vp3/1-69-H6 is shown in Figure 4b. The proteinaceous substance NLS-vp3/1-69-H6 was purified and produced as has been described for the above mentioned histidine-tagged apoptin (vp3) proteins.

Human Saos-2 cells were micro-injected with NLS-vp3/1-69-H6 protein as described for the above-mentioned micro-injected proteinaceous substances. As negative control, the human tumor cells were micro-injected with the non-apoptotic protein MBP and as positive control MBP-vp3 protein was micro-injected. Within 6 hours after micro-injection, the majority of the Saos-2 cells containing both MBP-vp3 and NLS-vp3/1-69-H6 protein became apoptotic, whereas the cells containing MBP protein did not. Comparable results were obtained with NLS-VP3/1-80-H6.

Therefore, we conclude that proteinaceous substances containing the complete apoptin protein sequence or a specific apoptin protein fragment can induce apoptosis in human tumor cells.

### 4.6. Biological activity of MBP-VP3-66-121 in tumor and normal cells.

*pMalTBVp3dN66, MBP fusion of C-terminal 55 residues of Apoptin (MBP-Apoptin(66-121).* The C-terminal domain of Apoptin (ORF bp 196-363) was cloned in pMalTB at BamHI and SalI. The protein was expressed and purified as described for the MBP-vp3 protein. The protein is called MBP-vp3-66-121.

In order to determine whether additional tumor-specific fragments of Apoptin could be generated, we made and tested MBP-vp3-66-121 in microinjection/immunofluorescence experiments in Saos-2 tumor cells and low-passage CD31-human normal dermal fibroblast cells exactly as described previously. MBP-vp3, as a control, induced tumor-specific nuclear localization and death in Saos-2 but not CD31-fibroblasts. Interestingly, the MBP-vp3-66-121 protein behaved very similarly to the full-length protein both in function as well as in specificity.
These results indicate that a C-terminal fragment of Apoptin behaves as a functional fragment of the full length Apoptin: the fragment is capable of killing tumor cells but does not induce apoptosis in normal cells.
These results suggest that protein fragments smaller than full-length Apoptin, and even peptides thereof, can be generated and used to achieve tumor-specific killing with no side effects in normal cells.

### 5. TAT-apoptin

### 5.1 Transduction using denatured and refolded apoptin protein

### 5.1.1 Description of transduction-domain-apoptin construct

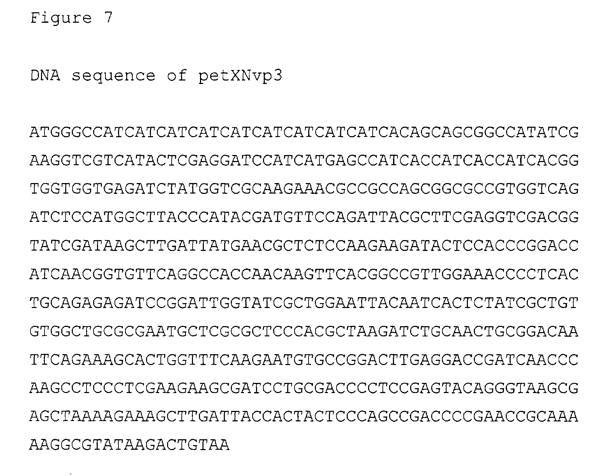

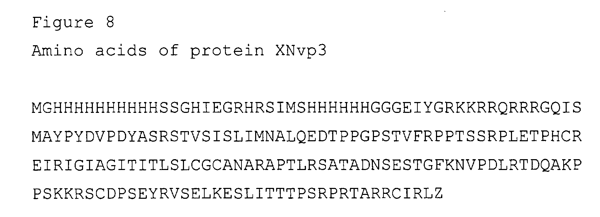

A DNA was constructed that encodes in frame for respectively a 6xHis-tag, a TAT-transduction domain, an HA-tag, followed by the coding sequence for apoptin and a stop codon. This construct was cloned in frame in a pet16b (Novagen) expression vector, which encodes for a 10x His-tag followed by the insert. The resulting construct is referred to as pETXNvp3, and the resulting protein will be referred to as XNvp3. See for the DNA sequence, figure 7 and for the protein sequence, figure 8.

### 5.1.2 Description of expression and purification

pETXNvp3 plasmid DNA was transformed into BL21(DE3)pLysS bacteria (Novagen) on LB plates containing the appropriate antibiotics, and an antibiotic resistant colony was grown to OD₆₀₀ of 0.6 in 1 liter LB plus antibiotics in a shaker flask. IPTG was added to 1 mM, and the cells were grown for an additional 3 hrs.

A bacterial pellet was obtained by centrifugation and sonicated on ice in 30 ml lysis buffer containing 150 mM NaCl and 0.5% Triton. After centrifugation the pellet was again sonicated as above, and inclusion bodies were centrifuged as a pellet. Expression of XNvp3 was confirmed by SDS-PAGE followed by Coomassie-blue staining and Western-blot analysis with 111.3, a vp3-protein specific monoclonal antibody.

The inclusion bodies were re-suspended in Nickel Loading Buffer (NLB;8M Urea, 1M NaCl, 50mM Phosphate buffer pH 7.5, 40mM imidazole) and loaded onto a 4 ml Ni-NTA column (Qiagen) equilibrated in the same buffer. The column was washed with 30 ml NLB, and washed with 10 ml MonoS Loading Buffer (MSLB;8M Urea, 250mM NaCl, 50mM phosphatebuffer pH 7.5) supplemented with 40mM imidazole. The bound proteins were eluted with MSLB supplemented with 250mM imidazole. The protein containing fractions were pooled and subsequently loaded on a 5 ml Mono-S chromatography column (Pharmacia) and washed with 2 column volumes MSLB. Refolding and elution was performed by washing the Mono-S column with 2M NaCl, 50mM phosphate buffer pH 7.5. The protein containing fractions were pooled and the buffer was exchanged on a PD10 column (Pharmacia) against DMEM or PBS. The protein was aliquoted and frozen at -80°C until further use.

### 5.1.3 Description of in-vitro transduction and specific killing of tumor cells

### Intracellular localization in tumor cells and normal cells.

To show that XNvp3 was able to penetrate into cells, and to assess its intracellular localization, the following experiment was performed. XNvp3 protein was added to cultured Saos-2 cells and VH10 cells at 50nM concentration in medium. After one hour the cells were fixed with 80% acetone, and the intracellular presence of XNvp3 protein was tested using antibodies 12CA5 (directed against the HA-tag) or 111.3 antibodies (directed against vp3 protein).

In both tumor and normal cells XNvp3 was located in the nucleus, as seen with confocal laser microscopy. This shows that XNvp3 can transduce into cells.

### Binding to Apoptin Associated Proteins in COS cells.

Next, we examined whether XNvp3 protein can bind to apoptin associated proteins as has been reported for apoptin protein (Noteborn and Danen-van Oorschot, 1999). To that end, the following experiment was performed. XNvp3 protein was incubated with COS cells that had been transfected 48 hours earlier with expression vectors encoding myc-tagged AAP1, which is one of the apoptin-associating proteins, or LacZ. Subsequently, the cells were lysed and an immunoprecipitation (IP) was performed with 12CA5. As a negative control, COS cells transfected with the same expression vectors but not incubated with XNvp3 were treated the same way. As a positive control, COS cells transfected with the same expression vectors together with an expression vector for HA-tagged vp3 were treated the same way. The IP's were analyzed for the presence of the myc-tagged proteins by SDS-PAGE and Western blotting with 9E10 (against the myc-tag).

The results show that XNvp3 can bind to an intracellular AAP in a similar fashion as apoptin (vp3) encoded by a transfected plasmid. Therefore, one can conclude that apoptin proteinaceous substances reveal essential biological activities of apoptin i.e. binding to its cellular counterparts.

### Induction of apoptosis in tumor cells versus normal cells

To show that XNvp3 can induce apoptosis in tumor cells, Saos-2 and VHSV-40 cells were cultured in the presence of 10 or 50nM XNvp3. To correct for possible degradation of XNvp3, the medium was replaced twice a day with fresh medium containing freshly thawed XNvp3. After four days the cells were fixed and the intracellular presence of XNvp3 and apoptosis were assessed with immuno-histochemistry as described before (Danen-Van Oorschot et al., 1997). The results show that XNvp3 can induce apoptosis in these cells at concentrations of around 10 to 50 nM.

A similar experiment was performed with non-transformed VH10 cells to establish the specificity of XNvp3. VH10 cells were cultured in medium containing 10 or 50 nM XNvp3, but also in a five time higher concentration at 250 nM. After four days, no significant apoptosis could be detected in the VH10 cells.
The same experiment was also performed with non-transformed keratinocytes and with primary mouse lymfocytes, and no apoptosis above background (medium alone) could be detected here either.

### Prevention of tumor cell growth in vitro

To extend the observation that XNvp3 can cause apoptosis specifically in tumor cells, Saos-2 cells, U2-OS cells, and VH10 cells were split 1:10 and cultured for two weeks in the presence of 50nM XNvp3 (replaced twice daily as described above) or in normal medium. The cells were then fixed with Methanol/acetic acid, and stained with Coomassie Blue. Although the cells in the control medium had all grown to confluency, the Saos-2 and U2OS cells treated with XNvp3 had all disappeared from the dish, indicating that they had undergone apoptosis. The VH10 cells treated with XNvp3 had grown to the same density as control treated VH10, showing that XNvp3 does not inhibit growth of non-transformed cells.

### 5.2 Transduction of non-denatured apoptin protein using cross-linked transduction domain peptides

### 5.2.1 Conjugation of TAT-peptide to MBP-vp3

MBPvp3 was purified as described for the above-mentioned micro-injection experiments. The protein was then chemically conjugated to synthetic TAT-peptide (aa 37-72, CFITKALGISYGRKKRRQRRPPQGSQTHQVSLSKQ) as described by Fawell et al. (1994). In short, the purified MBP-vp3 protein was activated with iodoacetamide, and desalted and concentrated. 4-(maleimidomethyl)-cyclohexanecarboxylic acid *N*-hydroxysuccinimide ester (SMCC) was added and after 30 minutes at room temperature the reaction was terminated by desalting on a G-25 column in 100 mM Na2HPO4 (pH 7.5). TAT peptide was added to the MBPbvp3-SMCC adduct and stored overnight at 4°C. The cross-linked conjugate was purified by size exclusion gel filtration and frozen at -80°C in small aliquots in the presence of 10% glycerol. A sample was analyzed by SDS PAGE using Coomassie-blue staining or Western blotting with antibodies raised against the TAT-peptide, or against vp3. The preparation was estimated to contain more than 50% conjugated MBP-vp3 protein, based on the relative intensity of the protein band with an apparent higher molecular weight on SDS-PAGE or the Western blot using anti-vp3 antibodies. The conjugated protein is referred to as MBP-vp3-TAT.

### 5.2.2. Description of in vitro transduction and specific killing of tumor cells

### Intracellular localization in tumor cells and normal cells.

To show that MBP-vp3-TAT was able to penetrate into cells, and to assess its intracellular localization, the following experiment was performed. MBP-vp3-TAT protein was added to cultured Saos-2 cells and VH10 cells at 5 microgram/ml concentration in medium. After 16 hours the cells were fixed by acetone fixation, and the intracellular presence of MBP-vp3-TAT was tested with antibodies directed against MBP (monoclonal mouse anti-MBP-clone R29; Zymed Laboratories, Inc. and polyclonal rabbit anti-MBP- clone C18/sc-808; Santa Cruz Biotechnology, Inc.) or 111.3 (reactive with vp3). In both tumor and normal cells MBP-vp3-TAT was located in the nucleus, as seen with confocal laser microscopy. This shows that MBP-vp3-TAT can transduce into cells.

### 5.2.3. Binding to Apoptin Associated Proteins in COS cells.

To show that MBP-vp3-TAT can bind to apoptin associated Proteins as well as transfected vp3 in cells, the following experiment was performed. MBP-vp3-TAT was incubated with COS cells that had been transfected 48 hours earlier with an expression vector encoding myc-tagged AAP-1 or LacZ. After 16 hours, the cells were lysed and anti MBP-vp3-TAT immunoprecipitation (IP) was performed with anti-MBP antibodies. As a negative control, COS cells transfected with the same expression vectors but not incubated with MBP-vp3-TAT were treated the same way. As a positive control, COS cells transfected with the same expression vectors together with an expression vector for HA-tagged vp3 were lysed and an IP was performed with anti-HA antibodies. The IP's were analyzed for the presence of the myc-tagged proteins by SDS-PAGE and Western blotting with 9E10 (against the myc-tag). The results show that MBP-vp3-TAT can bind to intracellular AAP's proving again that the apoptin proteinaceous substance contains the biological activity as seen for apoptin produced by transcription and translation of its DNA.

### 5.2.4. Induction of apoptosis in tumor cells versus normal cells

To show that MBP-vp3-TAT can induce apoptosis in tumor cells, Saos-2 and VHSV-40 cells were cultured in the presence of 1 or 5 microgram/ml MBP-vp3-TAT. To correct for possible degradation of MBP-vp3-TAT, the medium was replaced twice a day with fresh medium containing freshly thawed MBP-vp3-TAT. After four days the cells were fixed and the intracellular presence of MBP-vp3-TAT and apoptosis were assessed with immunohistochemistry as described before (Danen-Van Oorschot, 1997). The results show that MBP-vp3-TAT can induce apoptosis in these cells at concentrations of around 1 or 5 microgram/ml.

A similar experiment was performed with non-transformed VH10 cells to establish the specificity of MBP-vp3-TAT. VH10 cells were cultured in medium containing 1 or 5 microgram/ml MBP-vp3-TAT, but also in a five time higher concentration at 25 microgram/ml. After four days, no significant apoptosis could be detected in the VH10 cells.

The same experiment was also performed with non-transformed keratinocytes and with primary mouse lymphocytes, and no apoptosis above background (medium alone) could be detected here either.

### 5.2.5. Prevention of tumor cell growth in vitro

To extend the observation that MBP-vp3-TAT can cause apoptosis specifically in tumor cells, Saos-2 cells, U2-OS cells, and VH10 cells were split 1:10 and cultured for two weeks in the presence of 5 microgram/ml MBP-vp3-TAT (replaced twice daily as described above) or in normal medium. The cells were then fixed with Methanol/acetic acid, and stained with Coomassie Blue. Although the cells in the control medium had all grown to confluency, the Saos-2 and U2-OS cells treated with MBP-vp3-TAT had all disappeared from the dish, indicating that they had undergone apoptosis. The VH10 cells treated with MBP-vp3-TAT had grown to the same density as control treated VH10, showing that MBP-vp3-TAT does not inhibit growth of non-transformed cells.

### 6.1 Delivery of MBP-apoptin by chemically coupling to anti-prostate-specific membrane antibodies.

Prostate-specific membrane antigen (PSMA) is a membrane-bound glycoprotein that is highly restricted to prostatic epithelial cells. PSMA is increased in association with prostatic cancer, particularly in hormone refractory disease. For instance, the LNCaP prostate cancer cell line has an estimated 180,000 molecules of PSMA per cell on its surface. Devitt et al. (2000) developed the J591 monoclonal, which internalises the prostate cancer cell upon binding to PSMA. Therefore, we have examined the effect of chemically coupling of purified J591 monoclonal antibody to purified bacterially produced MBP-apoptin. The coupling was carried out as described for the conjugation of TAT peptide to MBP-apoptin (section 5.2.1).

Next, we studied the cell killing effect of the addition of the covalently linked J591-MBP-apoptin protein product into the medium (5 microgram per millilitre) of PSMA-positive LNCaP cells and, as control, in the medium of PSMA-negative Saos-2 cells. To correct for possible degradation of J591-MBP-apoptin, the medium was replaced twice a day with fresh medium containing freshly thawed J591-MBP-apoptin. After four days the cells were fixed and the intracellular presence of J591-MBP-apoptin and apoptosis were assessed with immunohistochemistry as described before (Danen-Van Oorschot, 1997). The results show that J591-MBP-apoptin can induce apoptosis in LNCaP cells, but not in Saos-2 cells at concentrations of around 5 microgram/ml. These results show that linkage of MBP-apoptin to a specific antibody, which can become internalized, results in a specific receptor-mediated uptake of (MBP)-apoptin and consequently in induction of apoptosis.

A similar experiment was performed with non-transformed normal human primary prostate epithelial cells to establish the tumor-specificity of J591-MBP-apoptin. The primary human prostate cells contain PSMA at their surface. They were cultured in medium containing 5 microgram/ml J591-MBP-apoptin, but also in a five times higher concentration at 25 microgram/ml. After four days, no significant apoptosis could be detected in these primary prostatic epithelial cells. Immunofluorescence analysis clearly showed that the primary prostate epithelial had taken up significant amounts of J591-MBP-apoptin protein.

These results show that although the primary prostate cells have taken up the J591-MBP-apoptin product, the apoptin part does not induce apoptosis as has been described for apoptin as well as for MBP-apoptin protein products. With other words, delivery to a cell via surface antigens of a protein product containing apoptin will result in induction of tumor-specific apoptosis.

Similar results were obtained with chemically coupling of single-chain Fv antibodies directed against HER2/neu (scFvHER; Wang et al., 2001)). HER2/neu has been implicated in the oncogenesis of human prostate cancer. Clinical studies have suggested that over expression of HER2 is one of the indicators of poor prognosis in prostate cancer treatment. The above-described LNCaP cells express besides PSMA also high levels of HER2 protein. Therefore, we incubated LNCaP cells with MBP-apoptin coupled chemically with scFvHER. Exposure of LNCaP cells to scFvHER-MBP-apoptin caused remarkable cell death. PC3M cells, lacking HER2 protein, did not undergo apoptosis upon treatment with scFvHER-MBP-apoptin protein. Addition of the chemically coupled scFvHER/MBP-apoptin products to the medium of primary human prostate cells also did not result in induction of apoptosis, which shows the safety of the approach of delivery of apoptin protein chemically coupled to a scFv molecule, which can deliver apoptin into the cell.

In conclusion, the data obtained with recombinant MBP-apoptin coupled to specific (single-chain) antibodies confirm the observation that coupling of a fluorescein moiety to (MBP)-apoptin does not negatively influence the tumor-specific activity of apoptin. Therefore, specific delivery of biological active (tumor-specific induction of apoptosis) (MBP-)apoptin protein to human tumor cells via coupling to specific antibody protein molecules forms the base for a novel anti-tumor therapy.

### 6.2 Construction and production of a single-chain scFvHER-MBP-apoptin.

A DNA plasmid was constructed that encodes in frame for scFvHER coding sequences followed by the coding sequence for MBP-apoptin. To that end, scFvHER sequences were cloned into the plasmid pMBP-vp3. The antibody moiety of the fusion protein was fused to the N-terminal part of MBP-apoptin via the linker peptide (Gly(4)Ser(6)). The final plasmid was called pscFvMBP-apoptin. Recombinant scFvMBP-apoptin was produced in BL21(DE3) bacteria and purified as described for MBP-apoptin (see above). Western-blot analysis using apoptin-specific polyclonal antibodies revealed the production of the expected fusion protein product.

To test the apoptosis activity of the recombinant scFvHER-MBP-apoptin product the following tissue culture experiments were carried out. To that end, the bacterially produced recombinant scFvHER-MBP-apoptin protein product was added into the medium (1-5 microgram per millilitre) of HER2-positive LNCaP cells and, as control, into the medium of HER2-negative PC3M cells. To correct for possible degradation of scFvHER-MBP-apoptin, the medium was replaced twice a day with fresh medium containing scFvHER-MBP-apoptin fusion protein.

After four days the cells were fixed and the intracellular presence of scFvHER-MBP-apoptin and apoptosis were assessed with immunohistochemistry as described before (Danen-Van Oorschot, 1997). The results show that scFvHER-MBP-apoptin can induce apoptosis in LNCaP cells, but not in PC3M cells. Addition of the scFvHER/MBP-apoptin fusion products into the medium of primary human prostate cells did not result in induction of apoptosis, which shows the safety of this apoptin fusion protein.

In conclusion, these results show that a bacterially produced recombinant scFv-MBP-apoptin fusion product can be obtained in a soluble and biologically active form without loss of apoptin's apoptosis activity and without the loss of the affinity of scFv components to its specific antigen.

### Description of the figures

Figure 1 shows the map of the MBP-vp3 fusion product inclusive the Asn-stretch and the thrombin cleavage site.

Figure 2 shows the partial DNA sequence of MBP-vp3. Underlined are the ATG-initiation codon of vp3 (apoptin) as well as the TAA-stop codon of vp3 (apoptin). The upstream sequences of the ATG-codon of vp3 are from the MBP construct showing that the apoptin sequence is in frame with the MBP sequence.

Figure 3 shows the protein sequence of MBP-vp3.

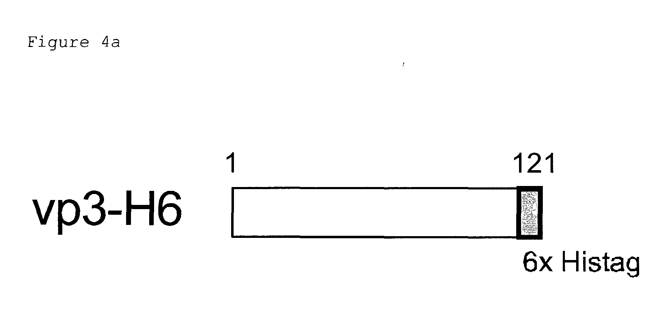

Figure 4a shows the map of vp3-H6, which is the complete apoptin (vp3) amino acid sequence containing 6 histidine residues at the C-terminal end.

Figure 4b shows the map of NLS-vp3/1-69-H6, which is made of the SV40 LT Nuclear Localization Signal (NLS), apoptin (vp3) amino-acid sequences 1-69 (1-69) and a C-terminal tag of 6 histidine residues.

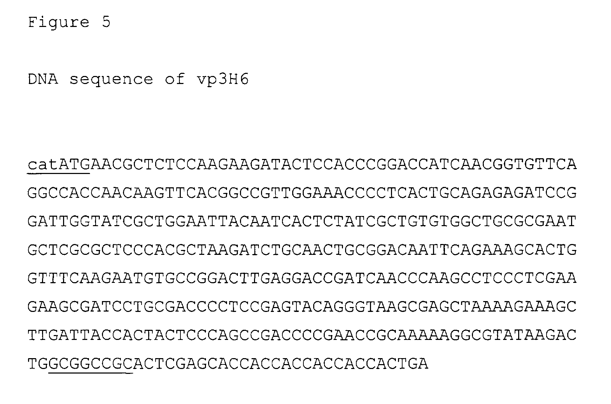

Figure 5 shows the DNA sequence of plasmid encoding the vp3H6 construct. The NdeI and NotI sites are underlined in the shown DNA sequence. The vp3- and histidine-sequences are cloned in the NdeI and NotI sites of plasmid pET22b.

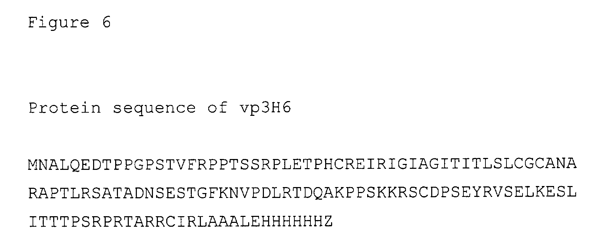

Figure 6 shows the protein sequence of the vp3H6 protein product.

Figure 7 shows the partial DNA sequence of pET-XNvp3 starting at the ATG of its NcoI site till the stop codon of apoptin (vp3).

Figure 8 shows the amino acid sequence of the XNvp3 protein product.

### REFERENCES

Antonsson, B., Montessiut, S., Sanchez, B. & Martinou, J. (2001). Bax is present as a high molecular weight oligomer/complex in the mitochiondrial membrane of apoptotic cells. J. Biol. Chem. 276(15), 11615-11623.
Bellamy, C.O.C., Malcomson, R.D.G., Harrison, D.J., and Wyllie, H. (1995). Cell death and disease: The biology and regulation of apoptosis. Seminars in Cancer Biology 6, 3-12.
Cain, K., Brown, D., Langlais, C. & Cohen, G. (1999). Caspase activation involves the formation of the Apoptosome, a large (700 kDa) caspase-activating complex. J. Biol. Chem. 274(32), 22686-22692.
Danen-Van Oorschot, A.A.A.M., Fischer, D.F., Grimbergen, J.M., Klein, B., Zhuang, S.-M., Falkenburg, J.H.F., Backendorf, C., Quax, P.H.A., Van der Eb, J.A., and Noteborn, M.H.M. (1997). Apoptin induces apoptosis in human transformed and malignant cells but not in normal cells. Proceedings National Academy Sciences, USA: 94, 5843-5847.
Danen-Van Oorschot, A.A.A.M, Den Hollander, A., Takayama, S., Reed, J., Van der Eb, A.J. and Noteborn, M.H.M. (1997a). BAG-1 inhibits p53-induced but not apoptin-induced apoptosis. Apoptosis 2, 395-402.
Derossi, D., Chassaing, G., and Prochiantz, A. (1998). Trojan peptides: the penetratin system for intracellular delivery. Trends in Cell Biology 8, 84-87.
Duke, R.C., Ocjius, D.M., Young, J, D-E. (1996). Cell suicide in health and disease. Scientific American December 1996, 48-55.
Elliott, G. and O'Hare, P. (1997). Intercellular trafficking and protein delivery by a herpesvirus structural protein. Cell 88, 223-233.
Earnshaw, W.C., 1995. Nuclear changes in apoptosis. Current Opinion in Cell Biology 7, 337-343.
Fawell, S., Seery, J., Daikh, Y., Moore, C., Chen, L.,.L., Pepinsky, B., and Barsoum, J. Tat-mediated delivery of heterologous proteins into cells. Proceedings National Academic Sciences USA 91, 664-668.
Frankel, A.D., and Pabo, C.O. 1988. Cellular uptake of the Tat protein from human immunodeficiency virus. Cell 55, 1189-1193.
Garzon-Rodriquez, W., Sepulveda-Becerra, M., Milton, S. & Glabe, C. (1997). Soluble amyloid Aβ-(1-40) exists as a stable dimer at low concentrations. J. Biol. Chem. 272(34), 21037-21044.
Green, M., and Loewenstein, P.M. 1988. Autonomous functional domains of chemically synthesized human immuno-deficiency virus Tat trans-activator protein. Cell 55, 1179-1188.
Hawiger, J. 1999. Non-invasive intracellular delivery of functional peptides and proteins. Current Opinion in Chemical Biology 3, 89-94.
Hockenberry, D.M. (1994). Bcl-2 in cancer, development and apoptosis. Journal of Cell Science, Supplement 18, 51-55.
Johnson, E., Evron, Y. & McCarty, R. (2001). Resonance energy transfer between tryptophan 57 in the e subunit and pyrene maleimide labeled g subunit of the chloroplast ATP synthase. Biochemistry 40, 1804-1811.
Kerr, J.F.R., Winterford, C.M., and Harmon, B.V. (1994). Apoptosis: Its significance in cancer and cancer therapy. Cancer 73, 2013-2026.
Lehrer, S. (1997). Intramolecular pyrene excimer fluorescence: a probe of proximity and protein conformational change. Meth. Enz. 278, 286-295.
Levine, A.J. (1997). p53, the cellular gatekeeper for growth and division. Cell 88, 323-331.
Lindgren,M. Haelbrink, M., Prochiantz, A., and Uelo Langel. Cell-penetrating peptides (2000). Trends in Pharmacological Sciences 21, 99-103.
McDevitt M.R., Barendswaard E., Ma D., Lai L., Curcio M.J., Sgouros G., Ballangrud A.M., Yang W.H., Finn R.D., Pellegrini V., Geerlings M.W. Jr, Le Brechbiel M.W., Bander N.H., Cordon-Cardo C., and Scheinberg D.A. (2000). An alpha-particle emitting antibody ([213Bi]J591) for radioimmunotherapy of prostate cancer. Cancer Research 60, 6095-6100.
McDonell T.J., Meyn, R.E., Robertson, L.E. (1995). Implications of apoptotic cell death regulation in cancer therapy. Seminars in Cancer Biology 6, 53-60.
Noteborn, M., Todd, D., Verschueren, C., de Gauw, H., Curran, W., Veldkamp, S., Douglas, A., McNulty, M., van der Eb, A. & Koch, G. (1994). A single chicken anaemia virus protein induces apoptosis. J. Virol. 68(1), 346-351.
Noteborn, M.H.M. (1996). PCT application WO 96/41191. Apoptin induces apoptosis in human transformed and malignant cells but not in normal cells as essential characteristic for the development of an anti-tumor therapy.
Noteborn, M.H.M., and De Boer, G.F. (1996). Patent USA/no. 030, 335.
Noteborn, M.H.M., De Boer, G.F., Van Roozelaar, D., Karreman, C., Kranenburg, O., Vos, J., Jeurissen, S., Zantema, A., Hoeben, R., Koch, G., Van Ormondt, H., and Van der Eb, A.J. (1991). Characterization of cloned chicken anemia virus DNA that contains all elements for the infectious replication cycle. Journal of Virology 65, 3131-3139.
Noteborn, M.H.M. and Danen-Van Oorschot. 1999. EP 99203465.2. AAP-1 in cytoplasma.
Noteborn, M.H.M., and Pietersen, A. (1998). A gene delivery vehicle expressing the apoptosis-inducing proteins VP2 and/or apoptin. PCT Application no. PCT/NL98/00213
Noteborn, M.H.M., Todd, D., Verschueren, C.A.J., De Gauw, H.W.F.M., Curran, W.L., Veldkamp, S., Douglas, A.J., McNulty, M.S., Van der Eb, A.J., and Koch, G. (1994). A single chicken anemia virus protein induces apoptosis. Journal of Virology 68, 346-351.
Noteborn, M.H.M., and Zhang, Y. (1998). Methods and means for determining the transforming capability of agents, for determining the predisposition of cells to become transformed and prophylactic treatment of cancer using apoptin-like activity. PCT Application no. PCT/NL98/00457
Noteborn, M.H.M., Danen-van Oorschot, A.A.A.M., Van der Eb, A.J. (1998a). Chicken anemia virus: Induction of apoptosis by a single protein of a single-stranded DNA virus. Seminars in Virology 8, 497-504.
Panse, V., Vogel, P., Trommer, W. & Varadarajan, R. (2000). A thermodynamic coupling mechanism for the disaggregation of a model peptide substrate by chaperone SecB. J. Biol. Chem. 275(25), 18698-18703.
Paulovich, A.G., Toczyski, D., Hartwell, H. (1997). When checkpoints fail. Cell 88, 315-321.
Pietersen, A.M., Van der Eb, M.M., Rademaker, H.J., Van den Wollenberg, D.J.M., Rabelink, M.J.W.E., Kuppen, P.J.K., Van Dierendonck, J.H., Van Ormondt, H., Masman, D., Van de Velde, C.J.H., Van der Eb, Hoeben, R.C., and Noteborn, M.H.M. (1999). Specific tumor-cell killing with adenovirus vectors containing the apoptin gene. Gene Therapy 6, 882-892.
Quiocho, F., Spurlino, J. & Rodseth, L. (1997). Extensive features of tight oligosaccharide binding revealed in high-resolution structures of the maltodextrin transport/chemosensory receptor. Structure 5(8), 997-1015.
Riddles, P. W., Blakeley, R. L. & Zerner, B. (1983). Reassessment of Elmann's reagent. Meth. Enz. 91, 49-60.
Sachs, L. and Lotem, J. (1993). Control of programmed cell death in normal and leukemia cells: New implications for therapy. Blood 82, 15-21.
Sahoo, D., Narayanaswami, V., Kay, C. & Ryan, R. (2000). Pyrene excimer fluorescence: a spatially sensitive probe to monitor lipid-induced helical rearrangement of apoplipophorin III. Biochemistry 39, 6594-6601.
Sanger, F., Nicklen, S., and Coulsen, A.R. (1977). DNA sequencing with chain-terminating inhibitors. Proceedings National Academic Sciences USA 74, 5463-5467.
Steller, H. (1995). Mechanisms and genes of cellular suicide. Science 267, 1445-1449.
Schwarze, S.R. Ho, A. Vocero-Akbani, A., and Dowdy, S. (2000). In-vivo protein transduction: delivery of a biologically active protein into the mouse. Science 285, 1569-1572.
Telford, W.G., King, L.E., Fraker, P.J. (1992). Comparative evaluation of several DNA binding dyes in the detection of apoptosis-associated chromatin degradation by flow cytometry. Cytometry 13, 137-143.
Teodoro, J.G. and Branton, P.E. (1997). Regulation of apoptosis by viral gene products. Journal of Virology 71, 1739-1746.
Thompson, C.B. (1995). Apoptosis in the pathogenesis and treatment of disease. Science 267, 1456-1462.
Vives, E., Brodin, P., and Lebleu, B. (1997). A truncated HIV-1 Tat protein basic domain rapidly translocates through the plasma membrane and accumulates in the cell nucleus. Journal of Biological Chemistry 272, 16010-16017.
Wang L., Liu B., Schmidt M., Lu Y., Wels W., Fan Z. (2001). Antitumor effect of an HER2-specific antibody-toxin fusion protein on human prostate cancer cells. Prostate 47, 21-28.
White, E. (1996). Life, death, and the pursuit of apoptosis. Genes and development 10, 1-15.
Wu, P. & Brand, L. (1994). Resonance energy transfer: methods and applications. Anal. Biochem. 218, 1-13. Wyllie, A.H. (1995). The genetic regulation of apoptosis. Current Opinion in Genetics and Development 5, 97-104.
Wyllie, A.H., Kerr, J.F.R., Currie, A.R. (1980). Cell death: The significance of apoptosis. International Review of Cytology 68, 251-306.
Zhuang, S.-M., Landegent, J.E., Verschueren, C.A.J., Falkenburg, J.H.F., Van Ormondt, H., Van der Eb, A.J., Noteborn, M.H.M. (1995). Apoptin, a protein encoded by chicken anemia virus, induces cell death in various human hematologic malignant cells in vitro. Leukemia 9 S1, 118-120.
Zhuang, S.-M., Shvarts, A., Van Ormondt, H., Jochemsen, A.-G., Van der Eb, A.J., Noteborn, M.H.M. (1995). Apoptin, a protein derived from chicken anemia virus, induces a p53-independent apoptosis in human osteosarcoma cells. Cancer Research 55, 486-489.

## Claims

1. Apoptin or functional equivalent or functional fragment thereof provided with a means to deliver apoptin to a cell.

2. Apoptin according to claim 1 capable of providing tumor-specific apoptosis.

3. Apoptin according to claim 1 or 2 wherein said means comprises a protein transduction system.

4. Apoptin according to anyone of claims 1 to 3 wherein said means comprises the HIV TAT protein.

5. Apoptin according to anyone of claims 1 to 4 further provided with a fusion protein or tag.

6. Apoptin according to anyone of claims 1 to 5 which is non-denatured.

7. A nucleic acid encoding apoptin according to anyone of claims 1 to 6.

8. A vector comprising a nucleic acid according to claim 7.

9. A host cell comprising a nucleic acid according to claim 7 or a vector according to claim 8.

10. A host cell according to claim 9 which is a prokaryotic cell such as E.coli.

11. Use of a proteinaceous substance comprising apoptin or functional equivalent or functional fragment thereof for induction of tumor-specific apoptosis.

12. Use of a proteinaceous substance according to claim 11 wherein said substance further comprises a means to deliver said apoptin to a cell.

13. Use of a proteinaceous substance according to claim 12 wherein said means comprises a protein transduction system.

14. Use of a proteinaceous substance according to claim 12 or 13 wherein said means comprises the HIV TAT protein.

15. Use of a proteinaceous substance according to anyone of claims 11 to 14 further comprising a fusion protein or tag.

16. Use of a proteinaceous substance according to anyone of claims 11 to 15 wherein said proteinaceous substance is non-denatured.

17. Use according to anyone of claims 11 to 16 wherein said apoptosis is p53-independent.

18. A pharmaceutical composition comprising a proteinaceous substance comprising apoptin or functional equivalent or functional fragment thereof capable of providing tumor-specific apoptosis.

19. A pharmaceutical composition according to claim 18 wherein said proteinaceous substance further comprises a means to deliver said apoptin to a cell.

20. A pharmaceutical composition according to claim 19 wherein said means comprises a protein transduction system.

21. A pharmaceutical composition according to anyone of claims 19 or 20 wherein said means comprises the HIV TAT protein.

22. A pharmaceutical composition according to anyone of claims 18 to 21 wherein said proteinaceous substance further comprises a fusion protein or tag.

23. A pharmaceutical composition according to anyone of claims 18 to 22 wherein said proteinaceous substance is non-denatured.

24. A pharmaceutical composition according to anyone of claims 18 to 23 wherein said apoptosis is p53-independent.

25. Use of a proteinaceous substance comprising apoptin or functional equivalent or functional fragment thereof capable of providing apoptosis for the preparation of a pharmaceutical composition for the treatment of a disease where enhanced cell proliferation or decreased cell death is observed.

26. Use according to claim 25 wherein said proteinaceous substance further comprises a means to deliver said apoptin to a cell.

27. Use according to claim 26 wherein said means comprises a protein transduction system.

28. Use according to claim 26 or 27 wherein said means comprises the HIV TAT protein.

29. Use according to anyone of claims 25 to 28 wherein said proteinaceous substance further comprises a fusion protein or tag.

30. Use according to anyone of claims 25 to 29 wherein said proteinaceous substance is non-denatured.

31. Use according to anyone of claims 25 to 30 wherein said apoptosis is p53-independent.

32. Use according to anyone of claims 25 to 31 wherein said disease comprises cancer or auto-immune disease.

33. A method for detecting the presence of cancer cells or cells that are cancer prone in a sample of cells comprising providing cells in said sample with a proteinaceous substance comprising apoptin or functional equivalent or functional fragment thereof and determining the percentage of apoptosis of cells in said sample.

34. A method for detecting the presence of cancer cells or cells that are cancer prone in a sample of cells comprising providing cells in said sample with a proteinaceous substance comprising apoptin or functional equivalent or functional fragment thereof and determining the intracellular localization of the proteinaceous substance in cells in said sample.

35. A method according to claim 33 or 34 wherein said proteinaceous substance further comprises a means to deliver said apoptin to a cell.

36. A method according to claim 35 wherein said means comprises a protein transduction system.

37. A method according to claim 35 or 36 wherein said means comprises the HIV TAT protein.

38. A method according to anyone of claims 33 to 37wherein said proteinaceous substance further comprises a fusion protein or a tag.

39. A method according to anyone of claims 33 to 38 wherein said proteinaceous substance is non-denatured.
